# EUROPEAN PATENT APPLICATION

(11) **EP 2 511 378 A1**
(43) Date of publication of application: **17.10.2012**
(21) Application number: 10835700.5
(22) Date of filing: 08.12.2010
(51) Int. Cl.: C12P 21/00, C12M 1/00, C12N 15/09

(54) **METHOD AND DEVICE FOR SYNTHESIZING PROTEIN FROM DNA MOLECULE CAPTURED IN MICROCHAMBER**

(30) Priority: 11.12.2009 JP 2009281673
(71) Applicant: Centre National de la Recherche Scientifique, 75794 Paris Cedex 16 (FR)
(72) Inventor: FUJII, Teruo, Tokyo 153-8505 (JP); KIM, Soo-Hyeon, Tokyo 153-8505 (JP); TAKEUCHI, Shoji, Tokyo 153-8505 (JP); FOURMY, Dominique, Tokyo 153-8505 (JP); YOSHIZAWA, Satoko, Tokyo 153-8505 (JP)
(74) Representative: Cabinet Plasseraud
(86) International application number: PCT/JP2010/007144
(87) International publication number: WO 2011/070776

(57) **Abstract**

Provided are a method and a device for synthesizing a protein from a DNA molecule captured in a microchamber, whereby the distance between microchambers can be shortened and thus the density of an array can be increased. Microchambers (32) are arranged at a high density. A DNA solution (34), which has been diluted so that one DNA molecule on average can be captured, is enclosed in the microchambers (32). Then, mRNA is synthesized using the one DNA molecule on average as a template. Based on this mRNA, a protein (37) is extracellularly synthesized.

## Description

### Field of the invention

The present invention relates to a method for synthesizing proteins from DNA molecules captured in microchambers, and the device used therefor.

### Background of the invention

In all cells, proteins are synthesized by processive enzymes called ribosomes. The dynamics of the translation process of genetic information carried by messenger RNA involving ribosomes remain to be completely elucidated (refer to Non-Patent Documents 1,2). Micrometer-size droplets and chambers are becoming extremely highly valuable tools in single-molecule enzymatic analysis procedures. Water-in-oil emulsions are used for the purpose of fractionating enzymes and gene libraries for protein synthesis (refer to Non-Patent Documents 3,4). Moreover, microchambers are also used in single-molecule enzymatic analysis (refer to Patent Document 1 and Non-Patent Document 5) and protein synthesis (refer to Non-Patent Document 6). Aqueous droplets of water-in-oil droplet emulsion are successfully applied to synthesize proteins from monomolecular DNA. Here, water-in-oil droplet emulsions are prepared in such a way that each droplet contains only DNA of no more than one molecule. In contrast, protein synthesis using microchambers lags far behind (refer to Non-Patent Document 6). Cases have been so far reported where extracellular proteins having a volume of about 100 mL were synthesized using arrays of polydimethylsiloxane (PDMS) microreactors and nano-well chips (refer to Non-Patent Documents 7 to 9). In addition, a technique has been developed with respect to low-capacity devices, wherein a green fluorescent protein (GFP) is synthesized by introducing in 1 pL PDMS chambers a gene DNA immobilized onto polymer beads (refer to Non-Patent Document 6), but which at least requires ten molecules of DNA per chamber to detect protein synthesis. Miniaturization of such microchamber arrays for protein synthesis would enable to construct protein chips at a very high density. Actually, methods to generate such protein chips by coupling extracellular protein synthesis and a DNA chip technology have been recently reported (refer to Non-Patent Documents 10 to 14). Protein chips are conventionally prepared by synthesizing proteins within viable cells and patterning the purified proteins in array format, but this methodology proved to be tedious and highly expensive.

### Prior Art Documents

### Patent Documents

Patent Document 1: Japanese patent No. 3727026

### Non-Patent Documents

Non-Patent Document 1: Andrei Korostelev and Harry F. Noller, [The Ribosome in Focus: New Structures bring New Insights], Trends in Biochemical Sciences, Vol. 32, No.9, pp. 434-441 (2007).
Non-Patent Document 2: R. Andrew Marshall, Collin Echeverria Aitken, Magdalena Dorywalska and Joseph D. Puglisi, [Translation at the Single-Molecule Level], Annual Review of Biochemistry, Vol. 77, pp. 177-203 (2008).
Non-Patent Document 3: Boris Rotman, [Measurement of Activity of Single Molecules of β-D-Galactosidase], Proceedings of the National Academy of Sciences of the United States of America, Vol. 47, pp. 1981-1991 (1961).
Non-Patent Document 4: Dan S. Tawfik and Andrew D. Griffiths, [Man-Made Cell-Like Compartments for Molecular Evolution], Nature Biotechnology, Vol. 16, pp. 652-656 (1998).
Non-Patent Document 5: Yannick Rondelez, Guillaume Tresset, Kazuhito V. Tabata, Hideyuki Arata, Hiroyuki Fujita, Shoji Takeuchi and Hiroyuki Noji, [Microfabricated arrays of femtoliter chambers allow single molecule enzymology], Nature Biotechnology, Vol. 23, pp.361-365 (2005).
Non-Patent Document 6: Takeshi Kinpara, Ryuta Mizuno, Yuji Murakami, Masaaki Kobayashi, Shohei Yamaura, Quamrul Hasan, Yasutsuka Morita, Hideo Nakano, Tsuneo Yamane and Eiichi Tamiya, [A Picoliter Chamber Array for Cell-Free Protein Synthesis], Journal of Biochemistry, Vol. 136, No.2, pp. 149-154 (2004).
Non-Patent Document 7: Takaoki Yamamoto, Takahiko Nojima and Teruo Fujii, [PDMS-Glass Hybrid Microreactor Array with embedded Temperature Control Device. Application to Cell-Free Protein Synthesis], Lab on a Chip, Vol. 2, pp. 197-202 (2002).
Non-Patent Document 8: Mari Tabuchi, Mami Hino, Yasuo Shinohara and Yoshinobu Baba, [Cell-Free Protein Synthesis on a Microchip], Proteomics, Vol. 2, pp. 430-435 (2002).
Non-Patent Document 9: Philipp Angenendt, Lajos Nyarsik, Witold Szaflarski, Jorn Glokler, Knud H. Nierhaus, Hans Lehrach, Dolores J. Cahill and Angelika Lueking, [Cell-Free Protein Expression and Functional Assay in Nanowell Chip Format], Analytical Chemistry, Vol. 76, No.7, pp. 1844-1849 (2004).
Non-Patent Document 10: Philipp Angenendt, Jurgen Kreutzberger, Jorn Glokler and Jord D. Hoheisel, [Generation of High Density Protein Microarrays by Cell-Free in Situ Expression of Unpurified PCR Products], Molecular & Cellular Proteomics, Vol. 5, No.9, pp. 1658-1666 (2006).
Non-Patent Document 11: Sheng-Ce Tao and Heng Zhu, [Protein Chip Fabrication by Capture of Nascent Polypeptides], Nature Biotechnology, Vol. 24, No.10, pp. 1253-1254 (2006).
Non-Patent Document 12: Niroshan Ramachandran, Jacob V. Raphael, Eugenie Hainsworth, Gokhan Demirkan, Manuel G. Fuentes, Andreas Rolfs, Yanhui Hu and Joshua LaBaer, [Next-Generation High-Density Self-Assembling Functional Protein Arrays], Nature Methods, Vol. 5, pp. 535-538 (2008).
Non-Patent Document 13: Mingyue He, Oda Stoevesandt, Elizabeth A. Palmer, Farid Khan, Olle Ericsson and Michael Taussig, [Printing Protein Arrays from DNA Arrays], Nature Methods, Vol. 5, pp. 175-177 (2008).
Non-Patent Document 14: Oda Stoevesandt, Michael J. Taussing and Mingyue He, [Protein Microarrays: High-Throughput Tools for Proteomics], Expert Review of Proteomics, Vol. 6, pp. 145-157 (2009).
Non-Patent Document 15: Paulo P. Amaral, Marcel E. Dinger, Tim R. Mercer and John S. Mattick, [The Eukaryotic Genome as an RNA Machine], SCIENCE, Vol. 319, No.28, pp. 1787-1789 (2008).

### Outline of the Invention

### Problems to be solved by the Invention

By contrast with aforementioned methodologies to generate protein arrays by synthesizing proteins in viable cells and printing them in array format, technologies consisting in synthesizing in situ proteins on arrays such as NAPPA (Nucleic Acid Programmable Protein Array) (refer to Non-Patent Document 12) and DAPA (DNA Array to Protein Array) (refer to Non-Patent Document 13) have been recently documented. Proteins are synthesized from genetic DNA spotted onto a first slide surface, while a tag is preliminarly arranged to bind onto a second slide surface. According to these methods which allow several thousands of proteins to be synthesized within an unpartitioned area, the synthesized proteins easily get spread and there is a potential risk for protein reciprocal contamination between spots. In order to solve this problem, with respect to the NAPPA method, attempts are carried out by affixing to a slide surface a tagged genetic DNA and introducing the tag in the C-terminus of a protein to localize in the vicinity of a corresponding gene the protein synthesized by means of an antibody capable to recognize and capture it. However, this method proved to be tedious and remains problematically impractical.

As regards the DAPA method on the other hand, measures are taken in an attempt to avoid such contamination by enlarging the distance between spots of genetic DNA (0.5 mm) and using a filter membrane to prevent proteins from spreading. In either approach however, usage of micro-fluid devices consisting of single compartments makes it very difficult to obtain spots of highly concentrated proteins from genetic DNA since spreading and dilution of synthesized proteins would occur, whilst the array density could hardly be increased due to a large inter-spot distance.

In view of such circumstances, the present invention has as its object to provide a method and a device for synthesizing proteins from DNA molecules captured in microchambers, whereby shortening of the distance between microchambers allows for increasing array density.

### Means for Solving the Problems

In order to achieve this purpose, the invention characteristically relates to:
[1] a method for synthesizing proteins from DNA molecules captured in microchambers, wherein microchambers are arranged at a high density, a DNA solution diluted to capture one DNA molecule on average is enclosed into microchambers, mRNA is synthesized using as a template said one DNA molecule on average, and synthesis of proteins is carried out extracellularly on the basis of this mRNA;

[2] the method for synthesizing proteins from DNA molecules captured in microchambers as mentioned in [1] above, wherein the microchambers refer to tiny chambers having a volume not exceeding 200 femtoliters;
[3] the method for synthesizing proteins from DNA molecules captured in microchambers as mentioned in [2] above, wherein the microchambers having a diameter of about 7 µm are arranged at distances corresponding to a center-to-center spacing of about 10 µm;

[4] the method for synthesizing proteins from DNA molecules captured in microchambers as mentioned in either [1] to [3] above, wherein the DNA solution is diluted 500-fold to give a bulk concentration of 8.0 pM versus a bulk concentration of 4.2 nM for usual reaction solutions;
[5] the method for synthesizing proteins from DNA molecules captured in microchambers as mentioned in either [1] to [3] above, wherein the DNA solution is diluted 400-fold to give a bulk concentration of 10.5 pM versus a bulk concentration of 4.2 nM for usual reaction solutions;

[6] the method for synthesizing proteins from DNA molecules captured in microchambers as mentioned in either [1] to [5] above, wherein the microchamber surface is subjected to surface modification by means of a polymer so as to restrain the adsorption of extracellular proteins thereto;
[7] the method for synthesizing proteins from DNA molecules captured in microchambers as mentioned in [6] above, wherein a MPC polymer [poly (2-methacryloyl oxyethyl phosphorylchloline-co-3-methacryloyl oxypropyl trimethoxysilane)] provides for the polymer;

[8] a device for synthesizing proteins from DNA molecules captured in microchambers, whereby protein arrays are generated at a high density and a high purity by extracellularly synthesizing proteins from one DNA molecule on average in each of the microchambers arranged at a high density;
[9] the device for synthesizing proteins from DNA molecules captured in microchambers as in [8] above, wherein each microchamber has a volume of not more than 200 femtoliters;

[10] the device for synthesizing proteins from DNA molecules captured in microchambers as in [9] above, wherein said microchambers having a diameter of about 7 µm are arranged at distances corresponding to a center-to-center spacing of about 10 µm;
[11] the device for synthesizing proteins from DNA molecules captured in microchambers as mentioned in either [8] to [10] above, wherein proteins are synthesized from one DNA molecule on average by introducing a diluted DNA solution into said microchambers.

[12] the device for synthesizing proteins from DNA molecules captured in microchambers as in [11] above, wherein said DNA solution is diluted 500-fold to give a bulk concentration of 8.0 pM versus a bulk concentration of 4.2 nM for usual reaction solutions;
[13] the device for synthesizing proteins from DNA molecules captured in microchambers as in [11] above, wherein said DNA solution is diluted 400-fold to give a bulk concentration of 10.5 pM versus a bulk concentration of 4.2 nM for usual reaction solutions;

[14] the device for synthesizing proteins from DNA molecules captured in microchambers as mentioned in either [8] to [13] above, wherein a polymer is provided to perform surface modification in said microchambers so as to restrain the adsorption of extracellular proteins thereto; and
[15] the device for synthesizing proteins from DNA molecules captured in microchambers as in [14] above, wherein the polymer is a MPC polymer [poly (2-methacryloyl oxyethyl phosphorylchloline-co-3-methacryloyl oxypropyl trimethoxysilane)].

### Effects of the Invention

According to the invention whereby proteins can be synthesized from one DNA molecule on average in micro-volume reactors (about 200 femtoliters) arranged at distances corresponding to a center-to-center spacing of about 0.01 mm, not only the purity of protein-encoding mRNA is guaranteed in each microchamber, but also protein arrays can be achieved at a high density (in principle, at least 2500 times that of the conventional devices).

### Brief Description of the Drawings

[FIG. 1] is a conceptual diagram of protein synthesis performed inside microchambers according to the invention.
[FIG. 2] presents SEM images of PDMS chip provided with microchambers for protein synthesis according to the invention.
[FIG. 3] is a graph depicting the variation with time in protein synthesis reactions of natural type GFP and EmGFP according to the invention.
[FIG. 4] allows for comparing GFP amounts synthesized within the microchambers according to the invention.
[FIG. 5] presents images indicating the variation with time in the course of reaction of the fluorescence microscope images of microchambers for protein synthesis during the EmGFP synthesis reaction according to the invention.
[FIG. 6] presents diagrams depicting the variation with time of the fluorescence intensity in microchambers for protein synthesis during the EmGFP synthesis reaction according to the invention.
[FIG. 7] presents diagrams depicting the statistical analysis of the protein synthesis reaction taking place in the microchambers according to the invention.
[FIG. 8] presents fluorescence images of microchambers according to the invention as taken after seven hours using specific filters for EmGPF and YFP.
[FIG. 9] is a conceptual diagram showing a high density PDMS chip provided with microchambers according to the invention.
[FIG. 10] is a conceptual diagram depicting a method to generate RNA chips by applying the method for synthesizing proteins according to the invention.
[FIG. 11] is a conceptual diagram depicting a method to generate protein chips using the method for synthesizing proteins according to the invention.

### Mode of Carrying out the Invention

The method for synthesizing proteins from DNA molecules captured in microchambers consists in setting up microchambers at a high density, enclosing thereinto a diluted DNA solution to capture one DNA molecule on average and synthesizing mRNA using this one DNA molecule on average as a template to perform extracellular protein synthesis on the basis of this mRNA.
Furthermore, the device designed for synthesizing proteins from DNA molecules captured in microchambers allows for preparing high density-high purity protein arrays by extracellularly synthesizing proteins from one DNA molecule on average in each of the microchambers from a plurality thereof arranged at a high density.

### Example

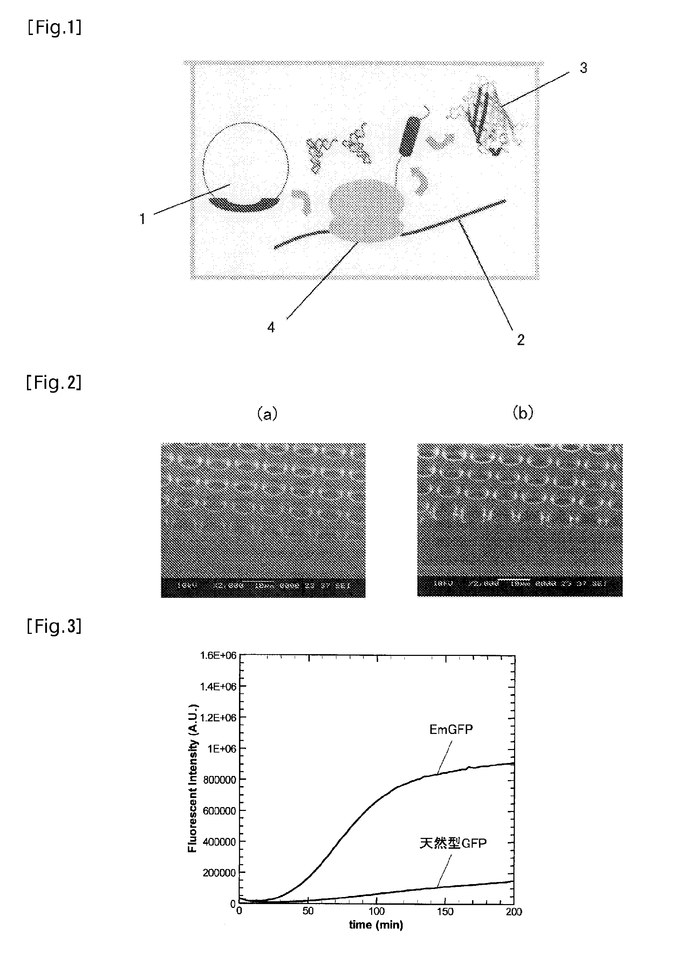

FIG. 1 shows a conceptual diagram of the protein synthesis performed inside microchambers according to the invention.
Synthesis of proteins is accomplished by coupling transcription and translation reactions. mRNA (2) is constructed by transcripting GFP-encoding DNA (1). The resulting mRNA (2) is translated to protein (GFP) (3) through vectors of the protein synthesis system. DNA (1) is bound to ribosome (4) to act as a template in protein synthesis. Here, the substance which is individualized inside the microchambers is a substrat for protein synthesis (DNA), but not an enzyme (ribosome to be specific). Each microchamber contains enough translational components (ribosomes, nucleotides, translational factors) to efficiently synthesize proteins, and these factors are not limitative.

According to the invention, the minimal gene DNA enables to easily synthesize proteins at a high purity in compartments consisting of microchambers using one DNA molecule on average. It is noteworthy that this method is kept free from any adherence of single DNA molecules onto the surface of the microchambers. Such DNA molecules remain in a state of free diffusion inside those microchambers.
This method enables to construct high-density chips having arrayed spots of highly concentrated proteins.

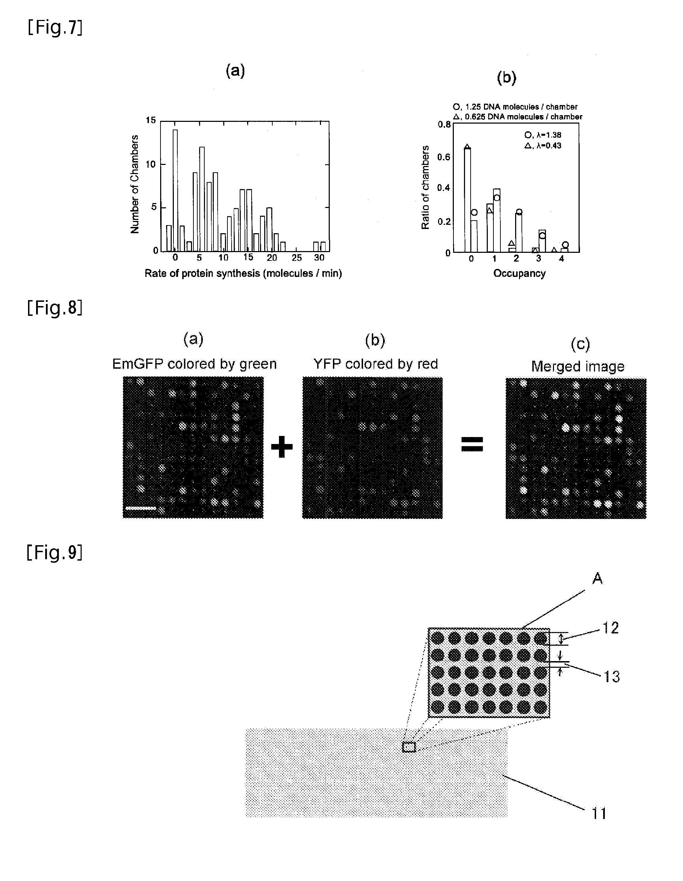

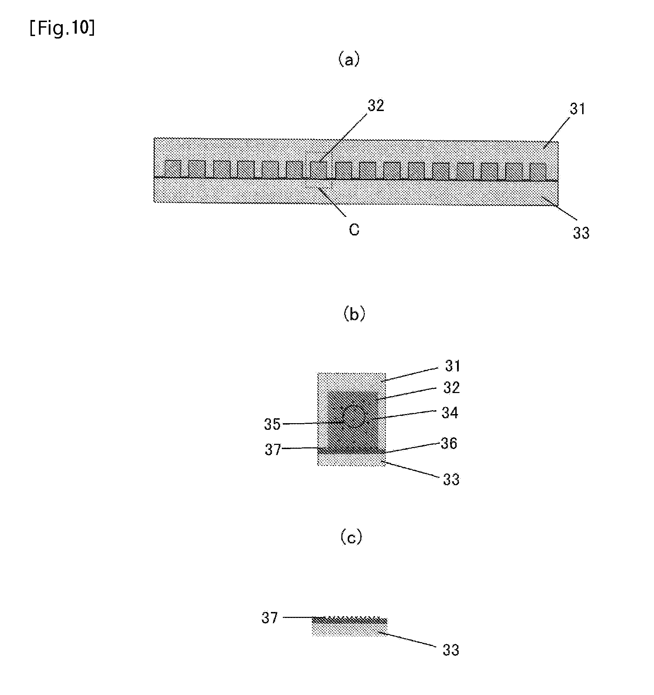

Firstly, a PDMS chip for protein synthesis was constructed. The PDMS chip and the template used for this purpose were generated by a commonly used replica molding technique and photolithography using a negative-type photoresist (SU-8 2005, MicroChem, Co.). Using as a template a cylindrically-patterned structure (7 µm in diameter, 5 µm in height) (refer to FIG. 8, FIG. 10), PDMS for microchambers was prepared. A fluorocarbon layer intended to ease PDMS withdrawal from the template was coated thereonto by CHF₃ plasma irradiation using a reactive ion etching system [RIE-10NR; Samco Inc.]. A mixture of polymeric precursor for PDMS [SILOPT 184; Dow Corning Toray Co., Ltd.] and a hardening agent in a weight ratio of 10 : 1 was poured over the template, which then was allowed to stand for thirty minutes in an oven heated at 110°C to cure the PDMS. Once peeled off from the template, the surface of the PDMS chip thus prepared was modified with 2-methacryloxy oxyethyl phosphorylcholine [MPC Polymer (Lipidure-CR1701 available from NOF Corporation)]. This modification prevents from protein adsorption onto the PDMS surface and allows for easing the introduction of reaction solution inside microchambers since PDMS surface turns to be hydrophilic (refer to Non-Patent Documents 7, 9).

FIG. 2 presents scanning electron microscope (SEM) images of the PDMS chip thus generated. FIG. 2(a) allows to observe at a 45 degrees angle the unmodified state, that is with no MPC coating and FIG. 2(b) the modified state, upon MPC coating.
The volume of each microchamber on this PDMS chip was about 190 femtoliters (fL). These SEM images indicate that a PDMS chip may be modified with a MPC polymer without breaking down the structure of any chamber. In order to prevent the reaction solution in the recipients from evaporating in the midst of the protein synthesis reaction process taking place in the microchambers, the MPC polymer-modified PDMS chip was soaked overnight in water and taken out therefrom just prior to usage.

With respect to extracellular synthesis of proteins, GFP or emerald GFP (EmGFP; Invitrogen) were synthesized using a commercially available kit (RTS 100 E. coli HY Kit, Roche Co.). This kit which contains all the factors necessary for in vitro protein synthesis was implemented according to directions for use. In bulk synthesis, the variation with time of the synthetized amount of proteins was quantified using a multilabel reader [Arvo (registered trademark) x 2; PerkinElmer, Inc.; excitation filter: 485 nm; emission filter: 535 nm]. The reaction solution was introduced in microchambers by inserting a droplet of solution for protein synthesis between the PDMS chip and a microscope cover glass. The removal of the solution in excess was carried out by pressing the PDMS chip with a blunt-tipped plastic rod, etc.. In this way, it was possible to hermetically-seal the solution inside each microchamber (refer to Patent Document 1 and Non-Patent Document 5). Hermetically-sealed microchambers were set onto the stage of an optical microscope and reacted at room temperature (25°C). Observations of how protein synthesis was going on were done using a high sensitivity electron multiplying charged coupled device (EMCCD) camera (iXon^{EM}+885 EMCCD Camera, Andor Technology Plc).

As a first step, bulk synthesis of natural type GFP and EmGFP was performed using a RTS100 Kit (reaction volume of 50 µL).
FIG. 3 is a graph depicting the variation with time in synthesis reaction of natural type GFP and EmGFP proteins.
Because of a slight gap between excitation and emission wavelengths of these proteins, no accurate comparison of protein synthesis amounts could be done with current experimental setups. However, as shown in FIG. 3, a major difference was identified between EmGFP and natural-type GFP. Basically, when a RTS100 system is employed, the synthesis rate of EmGFP is much faster than that of natural-type GFP, and efficiency is higher as well. These findings are well correlated with fast-occurring folding and maturation in EmGFP proteins.

Next, extracellular synthesis of proteins from one DNA molecule was performed using microchambers. A DNA solution with a concentration adjusted to 10.5 pM through a 400-fold dilution so that one DNA molecule on average fits into one microchamber was placed over the PDMS chip with the RTS100 Kit, and was hermetically sealed inside the microchamber according to the aforementioned method, then maintained at room temperature (25°C) to incubate.
Also, adjustement of the DNA solution through a 500-fold dilution to a concentration of 8.0 pM versus 4.2 nM for an usual bulk reaction solution enables to capture one DNA molecule per microchamber.

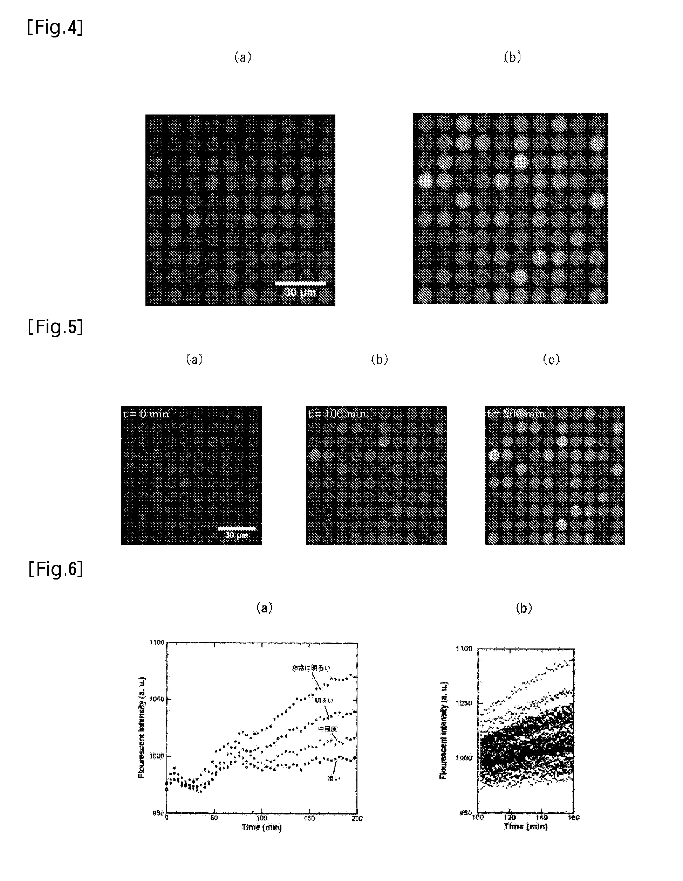

FIG. 4 presents fluorescence microscope images as taken 200 minutes after the reaction initiation to compare GFP amounts synthesized inside microchambers. FIG. 4(a) and FIG. 4(b) refer to cases where unmodified PDMS microchambers and a MPC coated PDMS chip were used, respectively.
As evidenced in FIG. 4, it was found that a more efficient protein synthesis is secured when the PDMS chip is subjected to MPC coating, as compared to the absence of such treatment. In addition, it turned out from these experimental results that the microchambers anyway require to be somehow surface-coated.

FIG. 5 shows the variation with time of fluorescence microscope images of microchambers for protein synthesis taken during the EmGFP synthesis reaction. Are indicated the variations occurred immediately after the reaction has started (FIG. 5(a)), 100 minutes later (FIG. 5(b)) and 200 minutes later (FIG. 5(c)). Besides, FIG. 6 illustrates the variation with time of the fluorescence intensity in microchambers for synthesizing proteins during the EmGFP synthesis reaction, FIG. 6(a) referring to a typical example of variation with time of the fluorescence intensity in the four classes of chambers, and FIG. 6 (b) indicating the variation with time of fluorescence intensity mean values in fifty randomly selected chambers in FIG. 5.

With reference to FIG. 5, there is a sign that the fluorescence intensity in microchambers gradually increase with each passing hour. As could be predicted from the fact that one DNA molecule on average was introduced in each microchamber, variations of the fluorescence intensity in microchambers did occur [refer to FIG. 5(b), (c)]. It might be seen that such variations of the fluorescence intensity in microchambers could be quantized in several classes (dark, moderately bright, bright, extremely bright).

The microchambers in FIG. 5 were sorted out into four classes (dark, moderately bright, bright, extremely bright) on the basis of fluorescence intensity and mean values of fluorescence intensity were determined for the respective classes. The variations with time of these values are shown in FIG. 6(a). The protein synthesis in microchambers showed a behaviour similar to that of the bulk synthesis in FIG. 3. The fluorescence signal from each microchamber increased over time to reach a plateau at 200 minutes. The synthesis rate of EmGFP in each class (graph slope) is distinctly different. As shown in FIG. 6(b) for the fifty randomly selected microchambers in FIG. 5, the variations with time of intensity fluorescence mean values in microchambers (from 100 minutes to 160 minutes after synthesis initiation) were plotted. The graphic gradient is in correspondence with the protein synthesis rate. The system can be calibrated by enclosing a solution of a known GFP concentration and plotting the fluorescence signals as a function of the number of contained molecules. This allows to estimate the number of GFP molecules which are synthesized every minute in the microchambers. The protein synthesis rate in microchambers exhibits several peaks quantized at equally-spaced intervals. These peaks reflect the protein synthesis rate from one molecule, two molecules, three molecules existing in a single microchamber, respectively.

FIG. 7 presents diagrams depicting the statistical analysis of the protein synthesis reaction in microchambers. FIG. 7(a) is a correlation chart between the variation gradient of the fluorescence intensity in all microchambers shown in FIG. 5 (100 microchambers) and the number of chambers with this gradient value. The five peaks correspond to the inclusion of 0 to 4 molecules of DNA into the microchambers, respectively. FIG. 7(b) shows the occupancy distribution in the case of 1.25 DNA molecule per microchamber [0 symbol] and in the case of 0.625 DNA molecule [0 symbol]. Here, O and Δ fit expected values. That is, they reflect the distribution of the DNA molecules per microchamber as determined in FIG. 7(a). These experimental results were found with a good Poisson distribution fitness.

The viability of this concept is demonstrated with reference to two different types of DNA molecules bearing the EmGPF gene and the yellow fluorescent protein (YFP) gene. The DNA molecules are respectively added to a cell-free protein synthesis system at a concentration of 0.31 DNA molecule per microchamber. The synthesis system having DNA molecules is incubated in PDMS microchambers. FIG. 8 presents fluorescence images of microchamber arrays taken after seven hours, using specific filters for EmGFP or YFP. Here, EmGFP is colored in green [refer to FIG. 8(a)] and YFP in red [refer to FIG. 8(b)]. On the merged image [refer to FIG. (c)], correspondingly to what they contain, EmGPF, YFP or both of them, the microchambers are colored in green, red and yellow, respectively. Bar scale represents 30 µm. As shown in these figures, spots of pure proteins can be obtained by mixing two different types of molecules. From this, it can be expected that spots of proteins would be obtained from a DNA library coding for a complete genome.

Protein synthesis was extracellularly performed from one DNA molecule on average in microchambers exhibiting a volume of 190 femtoliters. mRNA was synthesized using DNA as a template in microchambers, then proteins were synthesized on the basis of this mRNA. The present invention allows for efficiently synthesizing RNA and proteins from one DNA molecule on average as contained in a microchamber. According to the invention and as shown in FIG. 9, 1200 x 400 microchambers 12 were formed at a high density on a 12 mm by 4 mm PDMS chip. As shown in the enlarged part A of FIG. 9, the microchambers with a diameter of 7 µm 12 make up a PDMS chip 11 having a distance between adjacent microchambers 13 of 3 µm. Such dense arrangement of microchambers 12 allows for easily generating RNA chips and protein chips at a high density.

RNA molecules play an important role in signal transduction, structure, catalysis and genetic information control. Through genome analysis, it has been clarified that they code for approximately 1.5% of proteins in the human genome. 60 to 70% of RNA molecules do not code for proteins [refer to Non-Patent Document 5 above]. FIG. 10 is a conceptual diagram of the method for generating RNA chip that incorporates the present method for synthesizing proteins. FIG. 10(a) is a diagram showing a PDMS chip for constructing a RNA chip, FIG. 10(b) is an enlarged view of part B in FIG. 10(a) and FIG. 10(c) is a diagram showing a RNA chip as peeled off from the PDMS chip.

In FIG. 10, numeral 21 refers to a PDMS chip, 22 is a microchamber, 23 a RNA chip, 24 a solution for in vitro transcription, 25 a template DNA, 26 a cover glass surface treated to immobilize tagged synthesized RNA, and 27 stands for RNA immobilized onto the cover glass surface 26.

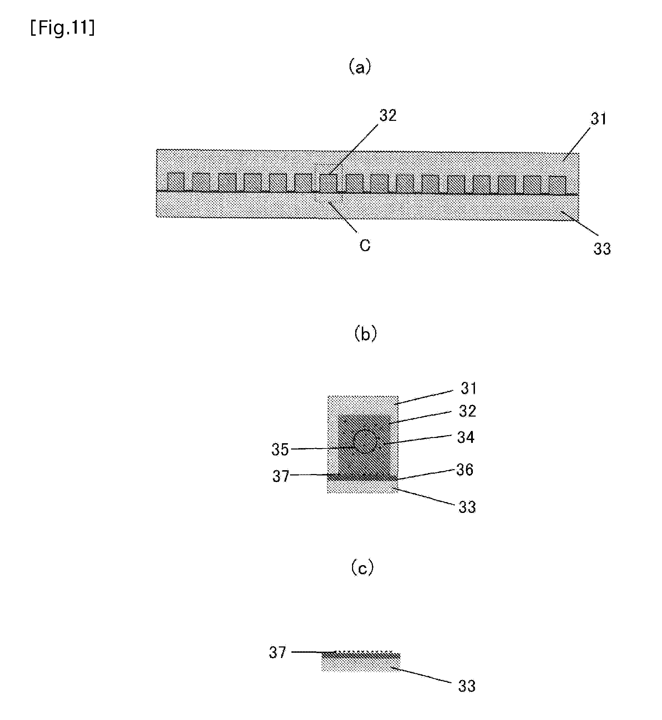

FIG. 11 is a conceptual diagram depicting a method to generate protein chips using the method for synthesizing proteins according to the invention. FIG. 11(a) is a diagram showing a PDMS chip for generating protein chips, FIG. 11(b) is an enlarged view of part C in FIG. 11(a) and FIG. 11(c) is a protein chip as peeled off from a PDMS chip.

In FIG 11, numeral 31 refers to a PDMS chip, 32 is a microchamber, 33 a protein chip, 34 a solution for extracellularly synthesizing proteins, 35 circular or linear DNA, 36 a cover glass surface treated to immobilize synthesized tagged proteins, and 37 stands for a protein immobilized onto the cover class surface 36.
The device in FIG. 11 is arranged so that the protein synthesis reaction is observed by means of an inverted fluorescence microscope (not shown) through a cover glass located in bottom part.

As aforementioned, usage of minuscule microchambers requires less expensive reagents for RNA and protein synthesis, thus enabling to curb at a large extent costs for generating RNA or protein chips. In principle, a tight arrangement of microchambers makes it possible to generate using a DNA library protein chips capable to produce at a high density proteins coded in one or several complete genomes on one chip of only a few square millimeters in size. In that way, drug screening tests and functional analysis of genes in one or several complete genomes could be easily performed. Furthermore, proteins (or RNA) associated with the method to construct protein (RNA) chips according to the invention would constitute a powerful tool for in vitro evolution systems if a mutated-gene library is employed.

The present invention is construed not to be limited to the aforementioned example and allows for various changes and modifications pursuant to its purpose, which are not excluded from the scope thereof.

### Industrial applicability

The method for synthesizing proteins from DNA molecules captured in microchambers and the device aimed at this purpose can be used as tools to synthesize proteins in a manner which does not require any large distance between microchambers and therefore that enables to highly densify arrays.

## Claims

1. A method for synthesizing proteins from DNA molecules captured in microchambers, **characterized in that** it consists in arranging microchambers at a high density, enclosing into microchambers a DNA solution diluted to capture one DNA molecule on average, synthesizing mRNA using as a template the one DNA molecule on average, and carrying out extracellular protein synthesis on the basis of this mRNA.

2. The method for synthesizing proteins from DNA molecules captured in microchambers according to claim [1], **characterized in that** the microchambers refer to tiny chambers having a volume not exceeding 200 femtoliters.

3. The method for synthesizing proteins from DNA molecules captured in microchambers according to claim [2], **characterized in that** the microchambers with a diameter of about 7 µm are arranged at distances corresponding to a center-to-center spacing of about 10 µm.

4. The method for synthesizing proteins from DNA molecules captured in microchambers as in any claim from [1] to [3], **characterized in that** the DNA solution is diluted 500-fold to give a bulk concentration of 8.0 pM versus a bulk concentration of 4.2 nM for usual reaction solutions.

5. The method for synthesizing proteins from DNA molecules captured in microchambers as in any claim from [1] to [3], **characterized in that** the DNA solution is diluted 400-fold to give a bulk concentration of 10.5 pM versus a bulk concentration of 4.2 nM for usual reaction solutions.

6. The method for synthesizing proteins from DNA molecules captured in microchambers as in any claim from [1] to [5], **characterized in that** the microchamber surface is subjected to surface modification by means of a polymer so as to restrain the adsorption of extracellular proteins thereto.

7. The method for synthesizing proteins from DNA molecules captured in microchambers according to claim [6], **characterized in that** a MPC polymer [poly (2-methacryloyl oxyethyl phosphorylchloline-co-3- methacryloyl oxypropyl trimethoxysilane)] provides for the polymer.

8. A device for synthesizing proteins from DNA molecules captured in microchambers, **characterized in that** protein arrays are generated at a high density and a high purity by extracellularly synthesizing proteins from one DNA molecule on average in each of the microchambers arranged at a high density.

9. The device for synthesizing proteins from DNA molecules captured in microchambers according to claim [8], **characterized in that** each microchamber has a volume of not more than 200 femtoliters.

10. The device for synthesizing proteins from DNA molecules captured in microchambers according to claim [9], **characterized in that** the microchambers with a diameter of about 7 µm are arranged at distances corresponding to a center-to-center spacing of about 10 µm.

11. The device for synthesizing proteins from DNA molecules captured in microchambers as in any claim from [8] to [10], **characterized in that** proteins are synthesized from one DNA molecule on average by introducing a diluted DNA solution into the microchambers.

12. The device for synthesizing proteins from DNA molecules captured in microchambers according to claim [11], **characterized in that** the DNA solution is diluted 500-fold to give a bulk concentration of 8.0 pM versus a bulk concentration of 4.2 nM for usual reaction solutions.

13. The device for synthesizing proteins from DNA molecules captured in microchambers according to claim [11], **characterized in that** the DNA solution is diluted 400-fold to give a bulk concentration of 10.5 pM versus a bulk concentration of 4.2 nM for usual reaction solutions.

14. The device for synthesizing proteins from DNA molecules captured in microchambers as in any claim from [8] to [13], **characterized in that** a polymer is provided to perform surface modification in the microchambers so as to restrain the adsorption of extracellular proteins.

15. The device for synthesizing proteins from DNA molecules captured in microchambers according to claim [14], **characterized in that** the polymer is a MPC polymer [poly (2-methacryloyl oxyethyl phosphorylchloline-co-3-methacryloyl oxypropyl trimethoxysilane)].
